(19) Europäisches Patentamt
European Patent Office
Office européen des brevets

(11) Veröffentlichungsnummer: **0 239 726 B1**

# (12) EUROPÄISCHE PATENTSCHRIFT

(45) Veröffentlichungstag der Patentschrift: **15.03.89**

(51) Int. Cl.⁴: **C07C 43/225**, C07C 79/35, C07C 41/01

(21) Anmeldenummer: **87100646.6**

(22) Anmeldetag: **19.01.87**

(54) **Verfahren zur Herstellung von Difluorchlormethoxybenzolen.**

(30) Priorität: **30.01.86 DE 3602681**

(43) Veröffentlichungstag der Anmeldung: **07.10.87 Patentblatt 87/41**

(45) Bekanntmachung des Hinweises auf die Patenterteilung: **15.03.89 Patentblatt 89/11**

(84) Benannte Vertragsstaaten: **BE CH DE FR GB IT LI NL**

(56) Entgegenhaltungen:
**EP-A- 0 052 558**
**EP-A- 0 168 344**
**EP-A- 0 196 529**
**EP-A- 0 199 660**
**US-A- 2 516 403**

**HOUBEN-WEYL "Methoden der organischen Chemie", Band E4: "Kohlensäure-Derivative", 1983, GEORG THIEME VERLAG, Stuttgart-New York, pp. 626-628, 1204**

(73) Patentinhaber: **BAYER AG, Konzernverwaltung RP Patentabteilung, D-5090 Leverkusen 1 Bayerwerk(DE)**

(72) Erfinder: **Marhold, Albrecht, Dr., Carl-Duisberg-Strasse 329, D-5090 Leverkusen 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft ein Verfahren zur Herstellung von Difluorchlormethoxybenzolen aus Phenolen.

Es ist bereits bekannt, daß man Difluorchlormethoxybenzole herstellen kann, indem man Trichlormethoxybenzole mit einem Fluorierungsmittel, z.B. Fluorwasserstoff oder Antimontrifluorid, stufenweise umsetzt (siehe Houben-Weyl, 4. Auflage, Band E4, Seite 628 (1983)).

Nachteilig hierbei ist, daß ein Ausgangsprodukt benötigt wird, das seinerseits in einem mindestens 2stufigen Verfahren aus Phenolen hergestellt werden muß, nämlich durch eine Methylierung und eine gegebenenfalls mehrstufige Seitenkettenchlorierung. Außerdem werden bei diesem Verfahren beim Einsatz z.B. von kernbromierten Methoxybenzolen die Kern-Bromatome bei der Chlorierung abgespalten und durch Chlor ersetzt, und Nitroanisole lassen sich überhaupt nicht an der Methoxygruppe chlorieren.

Weiterhin ist bekannt, daß man Phenole mit Tetrachlormethan und Fluorwasserstoff in Trifluormethoxybenzole überführen kann (siehe Houben-Weyl, 4. Auflage, Band E4, Seite 626 (1983)).

Für dieses Verfahren sind relativ hohe Temperaturen erforderlich. Ersetzt man hierbei das Tetrachlormethan durch Fluortrichlormethan, so stellt man fest, daß Fluortrichlormethan noch weniger reaktiv ist und noch höhere Temperaturen und/oder einen Katalysator erforderlich macht. Difluorchlormethoxybenzole können in beiden Fällen nur mit kleinen Ausbeuten erhalten werden.

Schließlich ist von Trichlormethyl-kohlensäure-esterchlorid, das im folgenden auch als Diphosgen bezeichnet wird, bekannt, daß es in Gegenwart von sauer reagierenden Substanzen schon bei 20°C in 2 Mol Phosgen gespalten wird und in Gegenwart von Aktivkohle in Kohlendioxid und Tetrachlormethan zerfällt (siehe Houben-Weyl, Band 4E, Seite 1204 (1983)). Diphosgen neigt also schon bei tiefen Temperaturen zum Zerfall.

Es wurde nun ein Verfahren zur Herstellung von Difluorchlormethoxybenzolen aus Phenolen gefunden, das dadurch gekennzeichnet ist, daß man Phenole mit Trichlormethyl-kohlensäure-esterchlorid in Gegenwart von Fluorwasserstoff umsetzt.

In das erfindungsgemäße Verfahren können beispielsweise solche Phenole eingesetzt werden, die der Formel (I) entsprechen

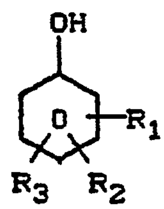

(I)

in der

$R_1$, $R_2$ und $R_3$ gleich oder verschieden sind und jeweils für Wasserstoff, Halogen, Alkyl, Aryl, O-Aryl oder S-Aryl stehen.

Vorzugsweise steht dabei einer oder zwei der Reste $R_1$ bis $R_3$ für Wasserstoff.

Bei Halogen kann es sich beispielsweise um Fluor, Chlor, Brom oder Iod handeln, vorzugsweise um Fluor, Chlor oder Brom. Bei Alkyl kann es sich beispielsweise um $C_1$- bis $C_4$-Alkyl handeln, vorzugsweise um Methyl. Aryl, O-Aryl und S-Aryl können beispielsweise 6 bis 8 C-Atome enthalten und im Arylteil gegebenenfalls substituiert sein, beispielsweise durch $NO_2$, CN, COHal (mit Hal beispielsweise Fluor), COOAlkyl (mit beispielsweise $C_1$- bis $C_4$-Alkyl), $SO_2F$, $SO_2Cl$, Alkyl (beispielsweise $C_1$- bis $C_4$-Alkyl), Halogen (beispielsweise Fluor, Chlor und/oder Brom), Alkoxy (beispielsweise $C_1$- bis $C_4$-Alkoxy) und/oder Halogenalkyl (beispielsweise Fluor-, Chlor- und/oder Brom-$C_1$- bis $C_4$-alkyl).

Besonders bevorzugt stehen $R_1$ und $R_2$ für Wasserstoff und $R_3$ für Methyl, Fluor, Chlor, Brom, $C_6$-Aryl oder O-$C_6$-Aryl, wobei die Arylgruppen gegebenenfalls einen der oben angegebenen Substituenten enthalten und sich der Rest $R_3$ in meta- oder para-Stellung zur phenolischen OH-Gruppe befindet, wenn es sich um Aryl oder O-Aryl handelt.

Ganz besonders bevorzugte Phenole der Formel (I) sind 4-Chlorphenol, 4-Bromphenol, 2-Chlorphenol, 2-Bromphenol, 2-Fluorphenol, 3-Fluorphenol, 4-Fluorphenol, 3-Bromphenol, 4-(4-Nitro-2,6-dimethyl-phenoxy)-phenol, 4-(4-Nitro-2,5-dimethyl-phenoxy)-phenol, 4-(4-Nitro-2-chlor-6-methyl-phenoxy)-phenol, 4-(4-Nitro-2-chlor-phenoxy)-phenol, 4-(4-Nitro-2-chlor-phenoxy)-2-chlorphenol, 4-(4-Bromphenyl)-phenol und 4-(4-Nitrophenyl)-phenol und 4-(4-Nitro-6-methyl-phenoxy)-phenol.

Das Diphosgen kann in handelsüblichen Qualitäten eingesetzt oder aus Ameisensäuremethylester und/oder Kohlensäuremethylesterchlorid durch Chlorierung hergestellt werden. Stöchiometrisch wird pro Mol eingesetztem Phenol 1 Mol Diphosgen benötigt. Man setzt deshalb im allgemeinen mindestens 1 Mol Diphosgen pro Mol Phenol ein. Vorzugsweise setzt man das Diphosgen aber im molaren Überschuß ein, beispielsweise 1,01 bis 5 Mol Diphosgen pro Mol Phenol.

Fluorwasserstoff wird im allgemeinen im Überschuß eingesetzt und kann gleichzeitig auch als Lösungsmittel für die erfindungsgemäße Umsetzung dienen. Beispielsweise kann man 5 bis 100 Mol Fluorwasserstoff pro Mol Phenol einsetzen. Vorzugsweise werden 8 bis 40 Mol Fluorwasserstoff pro Mol Phenol eingesetzt. Der Flurowasserstoff kann beispielsweise von technischer, wasserfreier Qualität sein.

Zusätzlich zu Fluorwasserstoff kann auch ein inertes Lösungsmittel eingesetzt werden, beispielsweise Dichlormethan, Dichlorethan, ein chlorierter Aromat, beispielsweise Chlorbenzol oder ein Reaktionsprodukt aus dem erfindungsgemäßen Verfahren.

Ebenfalls zusätzlich zu Fluorwasserstoff kann ein Friedel-Crafts-Katalysator, beispielsweise Titantetrachlorid zugesetzt werden.

Die Zugabe der Reaktanden und gegebenenfalls der Hilfsstoffe kann auf beliebige Weise und in beliebiger Reihenfolge erfolgen. Vorzugsweise sorgt man bei relativ tiefen Reaktionstemperaturen zu Beginn der Reaktion für einen Überschuß an Diphosgen, beispielsweise indem man das Phenol, gegebenenfalls zusammen mit Fluorwasserstoff, Lösungsmittel und/oder Katalysator, zu vorgelegtem Diphosgen hinzufügt. Bei relativ höheren Reaktionstemperaturen zu Beginn der Reaktion ist es bevorzugt, den Fluorwasserstoff, gegebenenfalls mit dem Phenol, dem Lösungsmittel und/oder dem Katalysator vorzulegen und Diphosgen hinzuzufügen oder Phenol und Diphosgen gleichzeitig in vorgelegten Fluorwasserstoff zu dosieren.

Das erfindungsgemäße Verfahren kann bei verschiedenen Temperaturen durchgeführt werden, beispielsweise bei −10 bis +100°C. Bevorzugte Temperaturen sind solche von 0 bis 60°C, besonders bevorzugt solche von 5 bis 50°C. Man kann das erfindungsgemäße Verfahren auch bei Raumtemperatur, z.B. 15 bis 25°C durchführen, und dabei auf Heizung und Kühlung verzichten. Man kann auch die Temperatur während der Reaktion verändern, z.B. die Reaktion bei −5 bis +20°C beginnen und bei einer gegenüber der Anfangstemperatur 5 bis 20°C höheren Temperatur zu Ende führen.

Die erfindungsgemäße Umsetzung ist im allgemeinen dann beendet, wenn keine Chlorwasserstoffentwicklung mehr beobachtet wird. Vorteilhafterweise hält man nach der Beendigung der Chlorwasserstoffentwicklung das Reaktionsgemisch noch einige Zeit auf Reaktionstemperatur. Geeignete Reaktionszeiten sind beispielsweise solche zwischen 1 und 30 Stunden.

Das danach vorliegende Reaktionsgemisch kann auf einfache Weise aufgearbeitet werden. Beispielsweise kann man dabei so verfahren, daß man zunächst überschüssigen Fluorwasserstoff und gegebenenfalls Lösungsmittel entfernt, beispielsweise durch Destillation. Den dabei verbleibenden Rest, aber auch das gesamte Reaktionsgemisch, kann man beispielsweise in Eiswasser austragen und den dabei sich bildenden Niederschlag abfiltrieren und gegebenenfalls weiter reinigen. Man kann auch den nach der Entfernung von überschüssigem Fluorwasserstoff und gegebenenfalls von Lösungsmittel verbleibenden Rest einer fraktionierten Destillation, vorzugsweise bei vermindertem Druck, unterwerfen.

Das erfindungsgemäße Verfahren hat z.B. die Vorteile, daß es die Herstellung von Difluorchlormethoxybenzolen bei niedrigen Temperaturen, aus gut zugänglichen Ausgangsprodukten und mit guten Ausbeuten und Selektivitäten gestattet.

Es ist ausgesprochen überraschend, daß diese Vorteile erzielt werden können, denn es war zu erwarten, daß das Diphosgen in Gegenwart von Fluorwasserstoff zersetzt wird, bevor es mit Phenolen reagieren kann.

Die gemäß dem erfindungsgemäßen Verfahren hergestellten Difluorchlormethoxybenzole können, auch ohne Isolierung aus dem Reaktionsgemisch, z.B. mit einem Fluorierungsmittel, wie Fluorwasserstoff, zu den entsprechenden Trifluormethoxybenzolen umgesetzt werden, aus denen durch Nitrierung, Reduktion und Umsetzung mit Isocyanaten Harnstoffe hergestellt werden können, die insektizid und/oder herbizid wirksame Stoffe sind (siehe z.B. EP-A 57 888, EP-A 93 976, EP-A 93-977, EP-A 132 680 und EP-A 122 449).

Beispiele

Allgemeine Arbeitsvorschrift

In einer Fluorierungsapparatur aus Edelstahl wurde Fluorwasserstoff vorgelegt, das jeweilige Phenol zugefügt und dann bei der angegebenen Temperatur Diphosgen unter Rühren zugetropft. Es setzte sofort eine starke Chlorwasserstoffentwicklung ein.

Nach dem Abklingen wurde zunächst überschüssiger Fluorwasserstoff, gegebenenfalls zusammen mit eingesetztem Lösungsmittel, durch Destillation bei Normaldruck zurückgewonnen. Anschließend wurde das Reaktionsprodukt unter vermindertem Druck abdestilliert.

Entsprechend dieser Arbeitsvorschrift wurden die in Tabelle 1 aufgeführten Einzelversuche durchgeführt.

Tabelle 1

| Beispiel Nr. | Menge HF | Eingesetztes Phenol | Menge Diphosgen | Reaktionszeit | Reaktionstemperatur | Reaktionsprodukt (-difluorchlormethoxybenzol) |
|---|---|---|---|---|---|---|
| 1 | 300 g | 4-Chlorphenol 60 g | 130 g | 9 h | 10–20°C | 4-Chlor- 56 g |
| 2 | 300 g | 4-Bromphenol 80 g | 275 g | 25 h | 10–20°C | 4-Brom- 77 g |
| 3 | 300 g | 2-Bromphenol 80 g | 130 g | 5 h | 20°C | 2-Brom- 48 g |
| 4 | 200 g + 100 ml $CH_2Cl_2$ | 4-(4-Nitro-2,6-dimethylphenoxy)-phenol 50 g | 80 g | 6 h | 20–50°C | 4-(4-Nitro-2,6-dimethylphenoxy)- 42 g |
| 5 | 400 g | 4-(4-Nitro-6-methylphenoxy)-phenol 100 g | 220 g | 15 h | 20°C | 4-(4-Nitro-6-methylphenoxy)- 67 g |

## Patentansprüche

1. Verfahren zur Herstellung von Difluorchlormethoxybenzolen aus Phenolen, dadurch gekennzeichnet, daß man Phenole mit Trichlormethyl-kohlensäure-ester-chlorid in Gegenwart von Fluorwasserstoff umsetzt.

2. Verfahren nach Anspruch 1, dadurch gekennzeichnet, daß man Phenole der Formel (I) einsetzt,

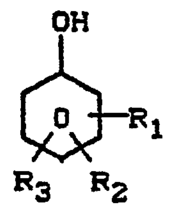

(I)

in der
$R_1$, $R_2$ und $R_3$ gleich oder verschieden sind und jeweils für Wasserstoff, Halogen, Alkyl, Aryl, O-Aryl oder S-Aryl stehen.

3. Verfahren nach Anspruch 2, dadurch gekennzeichnet, daß einer oder zwei der Reste $R_1$ bis $R_3$ für Wasserstoff stehen.

4. Verfahren nach Anspruch 2, dadurch gekennzeichnet, daß es sich bei Halogen um Fluor, Chlor, Brom oder Iod, bei Alkyl um $C_1$- bis $C_4$-Alkyl, bei Aryl, O-Aryl und/oder S-Aryl um 6 bis 8 C-Atome enthaltenden Arylreste handelt, die gegebenenfalls durch $NO_2$, CN, COHal, COOAlkyl, $SO_2F$, $SO_2Cl$, Alkyl, Halogen, Alkoxy und/oder Halogenalkyl substituiert sind.

5. Verfahren nach Ansprüchen 1 bis 4, dadurch gekennzeichnet, daß man pro Mol Phenol 1 bis 5 Mol Diphosgen einsetzt.

6. Verfahren nach Ansprüchen 1 bis 5, dadurch gekennzeichnet, daß man pro Mol Phenol 5 bis 100 Mol Fluorwasserstoff einsetzt.

7. Verfahren nach Ansprüchen 1 bis 6, dadurch gekennzeichnet, daß man zusätzlich zu Fluorwasserstoff ein inertes Lösungsmittel einsetzt.

8. Verfahren nach Ansprüchen 1 bis 7, dadurch gekennzeichnet, daß man das jeweilige Phenol zu vorgelegtem Diphosgen hinzufügt.

9. Verfahren nach Ansprüchen 1 bis 8, dadurch gekennzeichnet, daß man die Umsetzung bei Temperaturen von −10 bis +100°C durchführt.

10. Verfahren nach Ansprüchen 1 bis 9, dadurch gekennzeichnet, daß man Reaktionszeiten zwischen 1 und 30 Stunden einhält.

## Claims

1. Process for preparing difluorochloromethoxybenzenes from phenols, characterized in that phenols are reacted with trichloromethyl chloroformate in the presence of hydrogen fluoride.

2. Process according to Claim 1, characterized in that the phenols used have the formula (I)

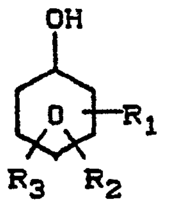

(I)

in which
$R_1$, $R_2$ and $R_3$ are identical or different and each stands for hydrogen, halogen, alkyl, aryl, O-aryl or S-aryl.

3. Process according to Claim 2, characterized in that one or two of the radicals $R_1$ to $R_3$ stand for hydrogen.

4. Process according to Claim 2, characterized in that the halogen is fluorine, chlorine, bromine or iodine, the alkyl is $C_1$- to $C_4$-alkyl, the aryl, O-aryl .and/or S-aryl are aryl radicals containing 6 to 8 C atoms which can be optionally substituted by $NO_2$, CN, COHal, COOalkyl, $SO_2F$, $SO_2Cl$, alkyl, halogen, alkoxy and/or halogenoalkyl.

5. Process according to Claims 1 to 4, characterized in that 1 to 5 moles of diphosgene are used per mole of phenol.

6. Process according to Claims 1 to 5, characterized in that 5 to 100 moles of hydrogen fluoride are used per mole of phenol.

7. Process according to Claims 1 to 6, characterized in that an inert solvent is used in addition to hydrogen fluoride.

8. Process according to Claims 1 to 7, characterized in that the particular phenol is added to initially introduced diphosgene.

9. Process according to Claims 1 to 8, characterized in that the reaction is carried out at temperatures of −10 to +100°C.

10. Process according to Claims 1 to 9, characterized in that reaction times between 1 and 30 hours are maintained.

**Revendications**

1. Procédé de fabrication de difluorochlorométhoxybenzènes au départ de phénols caractérisé en ce qu'on fait réagir des phénols avec du chloroformiate de trichlorométhyle en présence de fluorure d'hydrogène.

2. Procédé selon la revendication 1 caractérisé en ce qu'on utilise des phénols de formule (I),

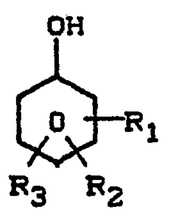

(I)

dans laquelle
$R_1$, $R_2$ et $R_3$ sont identiques ou différents et représentent respectivement l'hydrogène, un halogène, un alkyle, un aryle, un O-aryle ou un S-aryle.

3. Procédé selon la revendication 2 caractérisé en ce que un ou deux des restes $R_1$ à $R_3$ représentent l'hydrogène.

4. Procédé selon la revendication 2 caractérisé en ce que l'halogène est le fluor, le chlore, le brome ou l'iode, l'alkyle est un alkyle renfermant 1 à 4 atomes de carbone, l'aryle, l'O-aryle et/ou le S-aryle sont des restes aryle renfermant 6 à 8 atomes de carbone, qui sont éventuellement substitués par $NO_2$, CN, COHal, COOAlkyle, $SO_2F$, $SO_2Cl$, un alkyle, un halogéne, un alcoxy et/ou un halogéno-alkyle.

5. Procédé selon les revendications 1 à 4 caractérisé en ce qu'on utilise 1 à 5 moles de diphosgène par mole de phénol.

6. Procédé selon les revendications 1 à 5 caractérisé en ce qu'on utilise 5 à 100 moles de fluorure d'hydrogène par mole de phénol.

7. Procédé selon les revendications 1 à 6 caractérisé en ce qu'on utilise, en supplément au fluorure d'hydrogène, un solvant inerte.

8. Procédé selon les revendications 1 à 7 caractérisé en ce qu'on ajoute le phénol correspondant au diphosgène déjà présent.

9. Procédé selon les revendications 1 à 8 caractérisé en ce qu'on exécute la réaction à des températures de −10°C à +100°C.

10. Procédé selon les revendications 1 à 9 caractérisé en ce qu'on respecte des temps de réaction compris entre 1 et 30 heures.